# EUROPEAN PATENT SPECIFICATION

(11) **EP 2 010 116 B1**
(45) Date of publication and mention of the grant of the patent: **11.01.2012**
(21) Application number: 07724321.0
(22) Date of filing: 18.04.2007
(51) Int. Cl.: A61F 9/08

(54) **Compound subretinal prostheses with extra-ocular parts**
Zusammengesetzte subretinale Prothesen mit extraokularen Teilen
Prothèses sous rétiniennes composées avec parties extra-oculaires

(30) Priority: 21.04.2006 US 408852
(43) Date of publication of application: 07.01.2009
(73) Proprietor: Retina Implant AG, 72770 Reutlingen (DE)
(72) Inventor: BARTZ-SCHMIDT, Karl, Ulrich, 72070 Tübingen (DE); GRISANTI, Salvatore, 23654 Lübeck (DE); SZURMAN, Peter, 72070 Tübingen (DE); GEKELER, Florian, 72070 Tübingen (DE)
(74) Representative: Otten, Hajo
(86) International application number: PCT/EP2007/003384
(87) International publication number: WO 2007/121901

(56) References cited:
- EP-A- 1 618 922
- US-A1- 2004 098 067
- US-A1- 2005 251 223
- I. PETROU, E. ZRENNER: "Restoring sight to the bling" OPHTALMOLGY TIME EUROPE, October 2006 (2006-10), XP002436292

## Description

### Background of the invention

The present invention concerns a retinal implant to be implanted into an eye of a mammalian for electrical stimulation of the retina, said implant comprising an inner portion to be placed into the subretinal space, preferably on the retinal pigment epithelium, an extra-ocular portion that after implantation remains outside said eye for connection to an external power source, and a connecting portion for interconnecting said inner and extra-ocular portions.

For degenerative retinal diseases such as retinitis pigmentosa (RP) retinal prostheses represent one major potential treatment. With increasing numbers of human trials being performed retinal prostheses seem to be the nearest treatment option for patients suffering from these diseases at present; see e.g. US 6,761,724 to Zrenner et al., and US 5,024,223 to Chow.

Since 1995 there has been collected promising evidence of the feasibility of a subretinal prosthesis based on a microphotodiode array (MPDAs) which stimulates the retina from the subretinal space by transforming light energy into electrical energy. Evidence for the possibility to elicit spatially ordered responses in the cortex of animals is numerous and solid; see e.g. Eckhorn, et al. (2001), Physiological functional evaluation of retinal implants in animal models, Ophthalmologe 4:369-375; Gekeler et al. (2004), Subretinal electrical stimulation of the rabbit retina with acutely implanted electrode arrays, Graefes Arch Clin Exp Ophthalmol 7:587-596; and Schanze et al. (2005), Implantation and testing of subretinal film electrodes in domestic pigs, Exp Eye Res 82(2):332-40.

Questions concerning biocompatibility and biostability seem to a large extent be solved. There has also been presented evidence for the necessity to introduce extra energy in addition to the energy which subretinal photodiodes are capable to produce solely from transforming the light falling onto them into electrical energy; see e.g. Stett et al. (2000), Electrical multisite stimulation of the isolated chicken retina, Vision Res 40:1785-1795; and Stett et al. (1998), Electrical stimulation of degenerated retina of RCS rats by distally applied spatial voltage patterns, Invest Ophthalmol Vis Sci Abstract Annual Meeting.

This is in contradiction to other groups who believe subretinal implants consisting of MPDAs without external energy will produce enough electrical energy to stimulate the visual system; see Chow et al. (2004), The artificial silicon retina microchip for the treatment of vision loss from retinitis pigmentosa, Arch Ophthalmol 4:460-469.

Therefore, for the prosthesis of this application extra energy is crucial and currently two main different approaches are being investigated for introduction of this energy into the implant system: energy from infrared irradiation sources, as disclosed in US 6, 298,270, or energy transferred via high-frequency coils, as disclosed in US 6,847,947.

While these forms are wireless they are not yet readily available for human. Therefore, at present it is unavoidable to use subretinal prostheses with permanent extra-ocular connections to supply required additional energy.

Such prostheses are known, for example, from EP 1 618 922 A1, US 2004/0098067 A1 and US 2005/0251223 A1.

EP 1 618 922 A1 describes a retinal implant comprising an internal unit to be placed in a patient's eye, an external receiving unit and a cable connecting the two.

US 2004/0098067 A1 concerns a vision stimulating unit comprising a multiplicity of pixel circuits to be implanted in a patient's eye, a power supply circuit, located outside the patient's eye, and a cable connecting the power supply circuit with the pixel circuits.

US 2005/0251223 A1 discloses a retinal implant with a functional unit placed inside an eye, a functional unit positioned outside the eye, and a signal path connecting the functional units.

Humayun et al. (2003), Visual perception in a blind subject with a chronic microelectronic retinal prosthesis, Vision Res 24:2573-2581, report to have implanted epiretinal prosthesis with extra-ocular parts permanently into human volunteers. While the implant and the surgical procedure appear similar to the subretinal approach, several distinct differences exist: the subretinal implant used in this application and designed for human trials contained a much higher number of electrodes to elicit useful resolution (1550 vs. 16) and thus required a higher amount of additional energy; and epiretinal implantation techniques are easier from the viewpoint of the intraocular surgical procedure itself because they usually do not require extensive retinal surgery. To implant a compound system into the subretinal space and place external connections from there is the current challenge before human trials with this kind of prosthesis can be performed.

All previous studies with subretinal implants have only partially addressed and solved the surgical and technical problems arising from subretinal implantation of compound devices. They have been insufficient in not providing proof of the feasibility of the implantation of *compound* systems - from subretinal microphotodiodes to polyimide foils for choroidal access and silicone cables for transcutaneous energy supply; in addition, most of these experiments only acutely stimulated the visual pathways; see e.g. Sachs et al. (2005), Transscleral implantation and neurophysiological testing of subretinal polyimide film electrodes in the domestic pig in visual prosthesis development, J Neural Eng 1:S57-S64; and Schanze et al. (2005), Implantation and testing of subretinal film electrodes in domestic pigs, Exp Eye Res 82(2):332-40.

### Summary of the Invention

In view of the above, the object underlying the present invention is to improve the known subretinal implants.

The study underlying the present application has been proven the feasibility of long-term implantation of a new subretinal device which closely resembles the device for first human trials.

According to one object of the present invention, the implant is designed to allow stable fixation on the sclera and preferrably also on the skull; it is a compound system containing all parts necessary for long-term stimulation.

Further, it carries on its tip two different functional entities. First, an active MPDA of 1550 microphotodiodes, amplifiers, and electrodes (dimensions 3x3x0.1mm; array grid 70µm). It works autonomously and stimulates neighbouring neuroretinal tissue depending on relative light intensities. Second, a 4 x 4 array of electrodes spaced 280µm that can be addressed externally for direct stimulation (DS) of neuroretinal tissue. Charge injection delivered by each DS electrode can be controlled by a wireless stimulator, modulating amplitude, waveform, duration, frequency, and interstimulus intervals and allows highly controlled electrical stimulation in order define optimal pulses for subretinal electrical stimulation.

In this connection, surgical implantation technique has been altered in such a way that it now minimizes damage to the retina and the retinal pigment epithelium (RPE) and allows highly controlled introduction into the subretinal space to the exact required position. The feasibility of the approach has been proven by clinical examination (ophthalmoscopy, biomicroscopy), fluorescein angiography (FA), optical coherence tomography (OCT), and histology. Behavioural examination has demonstrated clear changes in animal behaviour following subretinal electrical stimulation.

The inventors succeeded in successful long-term implantation of a compound subretinal prosthesis with extra-ocular parts and energy coupling which represents the final step towards shortly forthcoming human trials.

The implant consisted of a microphotodiode array (MPDA) with 1.550 electrodes and a 4 by 4-array of gold electrodes for direct electrical stimulation; both were mounted onto a polyimide foil for transscleral placement into the subretinal space. The foil carried connection lanes to a silicone cable which was implanted under the skin and lead to a stimulator box in the animal's neck. Surgery was performed in eleven domestic pigs.

The new vitreo-retinal surgical technique comprises a 180° peripheral retinotomy and preferably use of diathermia to penetrate the choroid in order to avoid choroidal hemorrhage. This is contrary to the disclosure of US 6,761,724, that teaches a direct, transscleral, transchoroidal access to the subretinal region without opening the intraocular region to avoid operation risks of vitrectomy and retinotomy.

Subretinal forceps are used to place the implant safely onto the retinal pigment epithelium before the retina is flattened. Preferably, peripheral laser photocoagulation is applied and the eye is filled with silicon oil.

The implant is stabilized by a scleral fixation patch, and further preferably by use of a metal clamp with bone screws on the animal's skull and a tissue ring under the animal's skin in the neck.

All implants were successfully placed subretinally. In 3 animals a proliferative vitreo-retinopathy was observed after approximately 2 weeks. Otherwise, funduscopy and OCT demonstrated complete retinal attachment and FA showed no retinal vascular abnormalities over and around the implant. The animals showed clear behavioural reactions to electrical stimulation over the whole examination period. Histological examination failed to show any voltage-induced alteration in the cellular architecture of the retina overlying the stimulation electrodes.

This demonstrates the feasibility of the new surgical procedure for highly safe and controlled implantation of complex subretinal devices with extra-ocular parts.

### Brief Description of the Drawings

### FIGURE 1

Compound subretinal implant with microphotodiode array (MPDA) and 16 subretinal electrodes for direct stimulation (DS).
**a** The picture shows the compound system after explantation after four weeks. The implant closely resembles the implant for human trials. The plug (with the serial number) is connected to the remote-controlled power source for subretinal electrical stimulation, the silicone cable is placed subcutaneously in the animal's neck, the polyimide foil strip leads from the lateral canthus into the orbit and is fixed onto the sclera in the upper lateral quadrant of the eye. The tip of the foil strip carries the MPDA and the sixteen gold electrodes for DS. Overall length: ca. 35 cm, length of polyimide strip 10 cm, edge's length of MPDA 3 mm. Approximately 5 cm from the tip of the implant a patch for fixation on the sclera is shown in order to stabilize the subretinal part of the implant.
**b** The picture shows details of the polyimide foil and the tip with the MPDA and the electrodes for DS (spacing of the DS-array on the tip 280 µm, electrode dimensions 50 µm x 50 µm; the patch for scleral fixation is missing and the connection pad at the end is still unsoldered).

### FIGURE 2

Schematic diagram of the novel technique of implantation of subretinal compound systems.
**a** Standard 3 port-vitrectomy is performed with triamcinolone acetonide to visualize the posterior vitreous cortex.
**b** (1) After vitrectomy a fornix-based scleral flap is prepared with a size of 4 mm by 4 mm and a distance from the limbus of approximately 9 mm at the base. (2) The retina is detached by injection of balanced salt solution from the vitreous cavity using a subretinal cannula to create a localized bubble in the area of the scleral flap. (3) In the upper hemisphere of the eye a peripheral retinotomy is performed around 180° and approximately half of the retina is detached. During choroidal penetration diathermia is used to avoid choroidal hemorrhage.
**c** The implant can then be advanced into the subretinal space and exactly placed on the retinal pigment epithelium onto the desired position using subretinal forceps under fundus visualization.
**d** Perfluorodecaline is instilled to flatten the retina, the eye is filled with silicone oil and a circumferential peripheral laser photocoagulation is applied near the ora serrata. The scleral flap is then closed and the fixating patches of on the polyimide foil are attached to the sclera before the conjunctiva is closed.

### Detailed Description of a Preferred Embodiment

### Animals

Experiments were performed in 11 eyes of 11 domestic pigs. Pigs were obtained from a local breeder; they were cross-breedings of German Landrace and Piétrain. Experiments were performed in two series with 5 animals in each series. Animals weighed 60-110 kg. Animals were kept individually in crates without protruding edges where the animals could harm themselves or the external parts of the implant. All animal experiments adhered to the "Principles of laboratory animal care" (NIH publication No. 85-23, revised 1985), the OPRR Public Health Service Policy on the Humane Care and Use of Laboratory Animals (revised 1986) and the U.S. Animal Welfare Act, as amended, as well as the local commission for animal welfare.

### Anesthesia

General anaesthesia was induced by intravenous (IV) application of propofol (2.5-3.5 mg/kg; Disoprivan 2%®, AstraZeneca GmbH, Wedel, Germany) and fentanyl (0.02-0.15 mg/kg IV; Fentanyl-ratiopharm®, ratiopharm GmbH, Ulm, Germany). Animals were pressure-controlled ventilated (ventilation pressure: 18-20 cmH2O, tidal volume 5-10 ml/kg, frequency: 15-20/min, Fi=O2: 0.4). The balanced anaesthetic protocol combined the following drugs: (1) the hypnotic propofol (4-20 mg/kg/h), (2) the volatile anaestethic isoflurane (0.5-1.5 Vol%; Forene®, Abbott GmbH & Co. KG, Wiesbaden, Germany) and (3) fentanyl (0.03-0.1 mg/kg/h). Before implantation of the vena cephalica-catheter, muscle relaxation was induced by administration of pancuronium (0.06 mg/kg IV; Pancuronium Curared®, Curamed Pharma GmbH, Karlsruhe, Germany). Systemic hypotension, needed during penetration of the choroid, was reached depending on the clinical state by administration of either nitroprusside sodium (0.5-0.8 µg/kg/min IV; Nipruss®, Schwarz Pharma Deutschland, Monheim, Germany) or verapamil (0.05-0.1 mg/kg IV; Isoptin®, Abbott GmbH & Co. KG, Wiesbaden, Germany). Monitoring during anaesthesia included electrocardiography, pulse-oxymetry, non-invasive blood pressure measurement and clinical examination. During recovery, animals received the first intramuscular (i.m.) dose of enrofloxacin (2.5 mg/kg i.m.; Baytril 10%®, Bayer Vital GmbH, Leverkusen, Germany) and meloxicam (0.4 mg/kg i.m.; Boehringer Ingelheim Vetmedica GmbH, Ingelheim, Germany) to provide post-operative antibiosis and analgesia, respectively. Antibiosis was continued for 4 days, whereas length of analgesic treatment was determined by the clinical state (which didn't last more than 3 days in any animal). Follow-up examinations were carried out under propofol-sedation (2.5-3.5 mg/kg IV).

### The retinal prosthesis and subretinal electrical stimulation

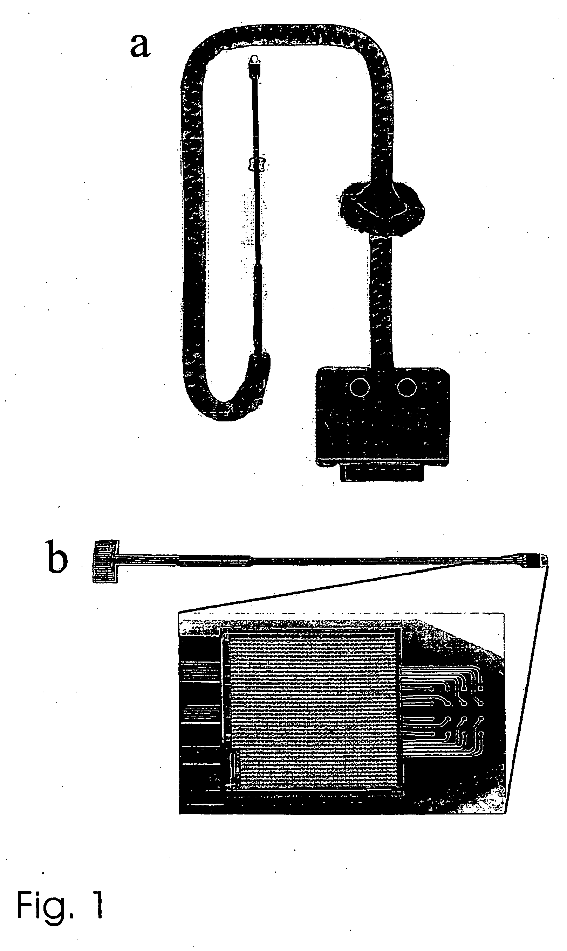

The device as manufactured by Retina Implant GmbH, Markwiesenstraße 55, D-72770 Reutlingen, Germany, consisted of a silicone cable (length ca. 25 cm) and a polyimide-foil strip (length 10 cm) carrying the light-sensitive MPDA with 1550 electrodes and an array of 16 gold electrodes for DS on the tip (figure 1). The MPDA was completely identical to MPDAs for later human use, including coating, electrode material and all electrical and mechanical connections. The 16 electrodes for DS (regular 4 x 4-spacing 280 µm, electrode dimensions 50 µm x 50 µm) were connected to the plug through golden connection lanes on the polyimide foil and twisted, insulated copper-wires within the silicone cable. The electrodes stimulated the retina by pulses generated within the box which was attached in the pocket in the neck of the pig. The box wirelessly received signals from a personal computer with a specially designed software program where pulses could be defined arbitrarily and individually for each electrode. Pigs were stimulated starting in the second week after implantation for 1 hour daily with different voltages between 0.5 V and 2 V (monophasic voltage-balanced pulses of 0.5 ms length, interstimulus interval 50 ms). Behavioural changes of the pigs were documented. Stimulation was performed in the exact same manner in each animal by the same examiners with stimulation voltages given in different sequence in order to avoid any additional clues to the animal which might influence their behaviour.

### Postoperative veterinary care

All pigs were monitored daily by animal care takers and veterinarians. Vital signs (heart rate, body temperature, food intake and animal behaviour) were recorded. All external wounds were inspected, thoroughly cleaned and antiseptic ointment was applied. Ophthalmic drops containing atropine and gentamycin were specially prepared and sprayed into the operated eye daily (University Hospital Pharmacy, Tübingen, Germany). At intervals of approximately 4-7 days the animals were put under short-term narcosis to allow inspection of the eye and the fundus and in order to take fundus pictures or allow OCT and FA examination.

### Surgical procedure

The surgical procedure can be divided into four steps which were carried out in the following order: (1) implantation of the venous catheter and (2) of the extra-ocular cable from neck to the orbit, (3) orbital and lid surgery, (4) vitreo-retinal surgery. The whole procedure required ventilation narcosis of approximately five to nine hours.

### Implantation of the venous catheter (1)

A permanent catheter was implanted into the right cephalic vein of each pig in order to allow safe and exact administration of drugs peri- and postoperatively. The catheter consisted of silastic tubing (Medical Grade Silicone Tubing, Espass 229, Route de Cannes, F-06130 Grasse, France; adapted to its final length during surgery according to the size of the animal). A disc of Dacron^{®} (Double Velour fabric, Cat. No. 007 827, Bard & DeBakey, Bard Angiomed, Karlsruhe, Germany) had been fixed previously in the middle of the catheter. The pig was placed in lateral recumbent position and a small incision was made anterior to the foreleg in the ventral cervical region. The external cephalic vein was exposed, a 2 to 3 cm segment of the vein was isolated for cannulation and a ligature was set distally to the segment. A small incision was made into the vein and the catheter was pushed forward to the brachiocephalic trunk and the anterior vena cava. The catheter was fixed in the vein using two ligatures. A specially-made trocar made of aluminum (3 mm x 15 mm x 500 mm) was used to tunnel the distal end of the catheter subcutaneously to the right side of the neck area, where it was exteriorized about 5 cm lateral to the dorsal midline of the pig. The catheter was anchored with a suture above the Dacron disc and the two incisions were closed with sutures.

### Subcutaneous placement of the cable (2)

The skin in the midline of the neck (in the region of the shoulder blades) was opened at a length of approximately 5 cm and the subcutaneous tissue was prepared into a depth of 2 cm. A custom-designed trocar made of aluminum was advanced from the anterior point of the wound to surface approximately 10 cm anteriorly. From there the skin was incised in an "S"-shaped manner to reach a point approximately 2 cm lateral to the lateral canthus. The incision was carefully deepened down and the skull was exposed. The anterior part of the implant was covered with a protective tubing, connected to the trocar and pushed forward to the frontal skin incision. Then the Dacron plate at the distal part of the implant was inserted into a circular subcutaneous incision and the overlying skin was closed with sutures. The implant was inserted into the frontal cut and fixed to a specially designed and manufactured clamp made from surgical stainless steel (length 3 cm, height 7 mm) which was screwed into the skull with two self-cutting bone screws (Synthes AG, Chur, Switzerland) to reduce tension on the more anterior delicate parts of the implant when the animals moved their heads and would thereby exert longitudinal forces onto to device. In most cases a third screw was inserted and the silicone tube of the implant was sutured to it with triple knots of a polyester thread (Mersilene 5-0^{®}, Ethicon, st-Stevens-Woluwe, Belgium).

### Orbital and lid Surgery (3)

The conjunctiva was opened near the limbus from the 8 over 12 to the 3 o'clock position and a subconjunctival canal was bluntly prepared to surface at the end of the "S" shaped cut near the lateral canthus. The tip of the implant could then be pulled through and positioned safely during the following vitreo-retinal procedure.

A fornix-based 50%-thickness scleral flap (4 mm x 4 mm) was prepared in 4 mm-limbus distance avoiding areas of vortex veins. The base of the flap was approximately 9 mm from the limbus.

### Vitreo-retinal surgery (4)

Vitrectomy was performed (Pentasys, Fritz Ruck GmbH, Eschweiler, Germany) using a wide field lens system (Ocular Instruments, Bellevue, WA). To reduce postoperative intraocular inflammation epinephrinhydrochloride (Suprarenine^{®} 1:1000, Aventis Pharma, Fraknfurt / Main, Germany), heparine (Liquemin 25.000^{®}, Roche, Grenzach-Wyhlen, Germany) and dexamethasone (DexaHEXAL 4 mg / 1 ml^{®}, Hexal, Holzkirchen, Germany) were added to the infusion solution as described by Szurman et al. (2005), Experimental implantation and long-term testing of an intraocular vision aid in rabbits, Arch Ophthalmol 7:964-969.

Triamcinolone acetonide (University Hospital Pharmacy, Tübingen, Germany) was used for better visualization of the posterior vitreous cortex. Balanced salt solution (BSS, Pharmacia, Groningen, Netherlands) was injected subretinally using a 41-gauge subretinal needle (Dual Bore BSS Injection Needle^{®}, 41 gauge 0.1 mm tip, MedTec, Mömbris, Germany) followed by a 180° retinotomy in the upper periphery. Subsequently, the subretinal space was accessed from externally in the area of the scleral flap using the diathermia needle of the machine in order to avoid choroidal bleeding and a 4 mm wide narrow opening. This maneuver was performed during systemic hypotension (see above) to further reduce the risk of choroidal bleeding. The implant was then advanced into the subretinal space for approximately 10 mm and the scleral flap was re-attached using 9-0 polygalactin sutures (Vicryl^{®}, Ethicon, St-Stevens-Woluwe, Belgium). From internally the device was pulled into the desired position under direct visualization using subretinal forceps. The retina was then re-attached using perfluorodecalin (HPF10^{®}, Al.chi.mi.a Europa, Ponte S. Nicolò, Italy) before performing the silicone oil exchange (Silicone Oil 5000 cps^{®}, Acri.Tec, Heningsdorf, Germany). A circumferential diode laser photocoagulation was applied near the ora serrata.

The polyimide foil was secured by suturing the fixation patches onto the sclera with 7-0 polygalactin (Vicryl®, Ethicon). The conjunctiva was finally carefully closed with single knots of 9-0 polygalactin sutures (Vicryl^{®}, Ethicon).

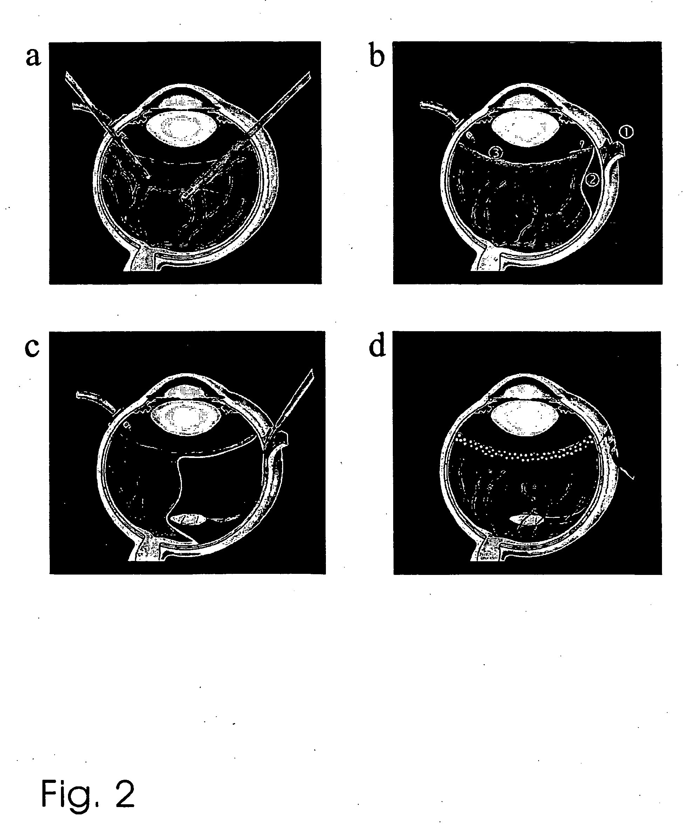

The surgical procedure is depicted schematically in figure 2.

### Fundus Photography and Fluorescein Fundus Angiography

Fundus photography was performed in all animals regularly with a hand-held fundus camera (Genesis^{®}, Kowa Company Ltd., Tokyo, Japan) on standard photographic slides. FA was performed in the standard manner as described (Hill et al. (1975), Infrared angiography of the cat fundus oculi, Arch Ophthalmol 2:131-133), in pigs of series one using a Scanning Digital Ophthalmoscope^{®} (Wild Medtec, Völkermarkt, Austria). Photographs were taken after injection of 10 mg/kg of 10% fluorescein into the venous catheter.

### Optical coherence tomography

OCT was performed in the standard manner in the first series as has been described before by Volker et al. (4-6-2004), In vivo assessment of subretinally implanted microphotodiode arrays in cats by optical coherence tomography and fluorescein angiography, Graefes Arch Clin Exp Ophthalmol 9:792-799. A STRATUS^{®} Optical Coherence Tomograph Model 3000 (Carl Zeiss Meditec, Dublin, CA, USA; software version 4.0.1) was used.

### Preparation for histology

After four weeks the animals were sacrificed and the implanted eyes immediately enucleated. After enucleation the anterior segment including the lens was removed and the posterior eye cups with the retina in place were fixed over night at 4°C in 4% paraformaldehyde in 100 mM phosphate buffer, pH 7.4. Following the fixation a fine slit was made through the retina directly in front of the implant and the device was carefully removed out of its tissue pocket. The eye cup was then embedded in paraffin by standard protocols; radial 5 µm sections were cut, collected on slides, and examined microscopically after staining with hematoxylin and eosin.

### Surgical results

Three of eleven animals died during recovery from general anaesthesia. Death was caused by cardiovascular breakdown resulting in cardiogenic shock, which is readily explained by bodyweight and breed of the used animals in connection with duration of general anaesthesia. To what extent the malignant hyperthermia syndrome (MHS) -which is a well known complication in the Piétrain breed- finally contributed to the lethal outcomes is unclear, although there were clinical signs which can be interpreted as slight symptoms of MHS. Probably bodyweight (and mainly the high percentage of muscle mass) together with long-lasting anaesthesia were the principal problems. One animal died during short-term narcosis for fundus photography on post-operative day 18. However, surgery could be successfully completed in all eleven animals (see below).

### Extra-ocular surgery

Extra-ocular surgery resulted in stable fixation of the implant in all cases throughout the observation period. The animals were freely moving in their stables and showed no apparent alteration in behaviour due to the mere presence of the implant (i.e. without electrical stimulation). Bending of their heads resulted in marked elongation of the silicone cable from the plug in the pocket to the fixation points on the bone of their forehead. The three extra-ocular fixation points prevented any movement of the delicate subretinal parts of the implant. All wounds were clean and occasional minor local infections in the stitch canals were successfully treated by local antibiotic ointment. In two animals of the first series the transition from the silicone cable into the polyimide foil was plastered with a bulkier silicone insulation which resulted in skin dehiscence near the lateral canthus that could be treated by placement of two additional skin sutures postoperatively. In the first series one animal suffered from conjunctival dehiscence which required re-suturing at two occasions. In the second series no such events were noticed.

### Vitreo-retinal surgery

In the first two animals of the first series a preliminary surgical procedure was performed (adopted from .the procedure introduced by Sachs et al. 2005, loc. cit.: first a small retinal bleb was created transvitreally by subretinal injection of BSS and access to the subretinal space was achieved from externally by choroidal penetration with a 2.75-mm keratome (Alcon, Fort Worth, TX, USA). In these first two animals there were encountered significant difficulties in arresting choroidal hemorrhages despite maximally tolerable systemic hypotension, local vasoconstriction and application of sodiumhyaluronat into the retinal bleb (Healon^{®}, Pharmacia, Erlangen, Germany). The bleeding also did not allow sufficient visualization of the device in the subretinal space for its secure positioning and placement was only controllable by external manipulation of the device. In these animals subretinal blood persisted in the follow-up period and interfered with fundus inspection and more importantly also with electrical stimulation. These difficulties prompted to switch to the described procedure with a peripheral 180° retinotomy and use of the subretinal forceps. While the first technique also resulted in localized mechanical RPE damage, no such changes were seen in the last nine cases. In addition, since no bleeding was observed during the choroidal penetration no subretinal blood was observed in the follow-up period. The final technique resulted in complete attachment of the retina in all cases.

In two animals of the last series lentectomy was performed. This resulted in considerable postoperative anterior chamber inflammation with formation of a fibrinous membrane on the lens capsule that obscured detailed fundus visualization. Neither the enhanced intra-ocular irrigation solution with heparin and dexamethasone nor daily subconjunctival injections of dexamethasone could successfully treat the inflammatory reaction. Another peculiarity of the pig eye was the markedly reduced corneal re-epithelialization in the cases where abrasion was performed during vitrectomy. Discontinuation of topical dexamethasone allowed slow recovery.

Major complication throughout the experimental series was a proliferative vitreo-retinopathy (PVR) which was observed in 3 animals between 10 to 14 days post-operatively and resulted in a slowly progressive tractional retinal detachment. In all other animals retinae remained completely attached during the whole examination period. Ophthalmoscopically no abnormalities of the retina or the RPE were observed, especially no bleeding, edema, neovascularization, atrophy, or epiretinal membrane formation were detected.

### Behavioral changes following subretinal electrical stimulation

In the stimulation experiments there was found a clear threshold dependence of the animals' behavioral changes. Below 1.0 Volts the animals did not show any behavioral changes. However, at or above 1.0 V all animals paused in their present action (i.e. eating, cleaning, sniffing, etc.) for approximately 2-5 seconds. Three pigs then clearly looked into the direction of the "visual field" of retinal stimulation (to the left since the electrodes were implanted into the superior-temporal region of the retina). In all other animals the reaction was marked, but the animals failed to show changes in the direction of gaze. Usually the animals started to continue in their prior action. All changes in behavior were highly reproducible and no attenuation was found.

### Optical coherence tomography and fluorescein angiography

Optical coherence tomography and fluorescein angiography were performable in the standard manner; see Volker et al. (4-6-2004), loc. cit. OCT revealed complete retinal attachment over and around the implant and also in the accessible area of the implantation channel. The retina was attached over the MPDA, the electrode array and the transition area between the two. FA showed totally intact vasculature without signs of leakage, obstruction or formation of new vessels in the area overlying the implant and surrounding it.

### Histology

Histology showed some abrasion of the PRs' outer segments that was due to the implantation process and partial disruption of the monolayered RPE in the area of the implantation channel. No changes, however, were observable in the cellular architecture of the retina overlying the stimulation electrodes of the implanted devices, except for the expectable degeneration of PRs' outer segments due to the interrupted nutrition from the RPE by the presence of the device in the subretinal space.

### Discussion

By use of a novel surgical technique and a new design of the implant with three additional extra-ocular fixation points there has been proven the feasibility of applying additional energy to implants having permanent extra-ocular connections.

The improved surgical technique was the major accomplishment in the study underlying this application. Choroidal penetration into the created subretinal space using diathermia provided a controllable and safe way for implantation by avoiding choroidal bleedings which has caused problems during surgery by obstructing visibility and also later by persisting subretinal blood which interfered with visualization of the implant and with electrical stimulation in previous studies and also in our first animal. Decreasing choroidal bleeding only by systemic hypotension and local vasoconstrictory agents proved insufficient. However, local diathermia avoided these bleedings. Retinal detachment over 180 degrees allowed placement of the implant in the subretinal space by use of subretinal forceps onto the exact desired RPE location (which seems absolutely necessary in view of heterogeneous fundus aspects of human candidates with RP) . In previous studies advancement of the device through the choroidal incision was uncontrollable because it was performed through a -more or less-bleeding choroidal opening and placement on the fundus was only controllable through movements of the external part of the implant. Gentle placement of the device avoided any RPE damage which has been observed before. RPE damage increases intraocular inflammatory responses and also -in cases where RPE cells come to lie between the implant and the retina- interfere markedly with electrical stimulation because separation of retinal target cells from the electrodes even by micrometers can lead to a massive increase in threshold.

While the new technique avoids these risks, it is clear that other risks have to be accepted because retinotomy over 180 degrees and transient retinal detachment infers a higher risk of developing PVR. In this study of 11 animals there have been observed 3 cases of PVR which corresponds to a rate of almost 30%. Contributing to that seems to be the pig model's inherent problems with developing inflammatory responses to any intraocular procedure. In this study, for example, simple and uncomplicated lentectomy led to an uncontrollable formation of a fibrinous membrane on the residual lens capsule and intraoperative corneal abrasion led to marked corneal edema and a (in relation to human standards) massively prolonged healing period. In addition, it is known from human ocular surgery that children tend to develop much stronger PVRs than adults at a much higher incidence of up to 70% following retinal detachment surgery. The pigs were studied during the period of rapid juvenile growth (age: 4.5 to 7 month; maximum expected life span: about 12 years) and had reached between 25 and 35% of the mature body weight at the time of surgery and thus showed a very high rate of metabolism. These facts and clinical experience with macular translocation surgeries, where usually a retinotomy is performed around 360 degrees, suggests that the PVR rate in first human trials will be much lower and -in case they develop- more accessible to repair. Using current techniques in macular rotation PVR rates are in the range of approximately 20%. As in macular rotation surgery for age-related macular degeneration there is currently no other accepted treatment option for RP other than highly experimental approaches and therefore a risk of 30% seems acceptable.

A second major improvement which led to stable fixation of the prosthesis in this study was the introduction of fixation points of the polyimide foil on the sclera. In previous studies it has been shown that polyimide is highly inert but smoldering inflammatory responses to it as a foreign body in the subconjunctival space led to walling-in of the material without firmly attaching to it. This led, especially with eye movements, to tractional forces on the implant with movements in the subretinal space which consequently imposed a high rate of retinal detachments and retinal injury. In contrast, no dislocation of the implant and neither retinal injury nor retinal detachment whatsoever were observed in our study. In all explantation procedures the implant proved to be well placed and no movement after four weeks was observed.

In an improvement there have been two patches at the polyimide or parylene foil for attaching the foil to two fixation points at the sclera. In order to further improve the fixation and to counter-act any slipping of the foil relative to the patches, the foil has been folded and bent by approx. by 90° between said two patches.

Scleral fixation alone, however, might not be enough without additional proper fixation of the implant at the point of cutaneous transit, which can e.g. be achieved on the skull with metal clamps and bone screws. These fixation points in combination with a highly flexible silicone cable between them avoided any movement of the subretinal part of the implant even in these highly active animals. This achievement was one major indispensable prerequisite for human implantations which will in a first step use an almost identical implant design. Problems, however, seem to be much less in adult human volunteers who will be able to control their movements and adhere to certain restricting rules.

In this study no endophthalmitis or other ocular infection was observed. Minor reddening or beginning infection in the extra-ocular stitch canals were easily treatable by antiseptic or antibiotic ointment. In humans the risk is obviously much lower due to higher hygienic standards. From maxillo-facial surgery it is known that transcutaneous implants are well tolerated for many years and therefore, no unacceptable risk of infection should be expected from the cutaneous transit. Permanent transscleral penetration, as for example in glaucoma drainage implants, imply a small risk of endophthalmitis which has been shown to lie at approximately 1.7%.

Optical coherence tomography has proven the ophthalmoscopic finding of good retinal attachment in the entire posterior pole and over the implant. Of special interest was the fact that the retina closely followed the device's surface in the transition from the thicker MPDA to the flatter electrode area for DS near the tip of the foil. This is of importance in stimulation of the DS-electrodes in humans which is planned for exact determination of thresholds and definition of optimal pulse designs for subretinal electrical stimulation.

Fluorescein angiography has demonstrated totally intact retinal vasculature over and around the implant in the posterior pole. No retinal edema, neovascularization, or vessel obstruction in the region of the implant, or over the stimulation electrodes or over the MPDA was observed in FA. This is the first report of intact retinal vessels and structure -as shown by OCT and FA- after prolonged subretinal electrical stimulation with supra-threshold voltage patterns.

To this day, there are numerous proofs of the feasibility of subretinal electrical stimulation to evoke spatially ordered responses in the animal brain, especially of pigs. But the pig skull presents specific problems in registering evoked cortical potentials because of the thickness of the bone which requires neurosurgical procedures to contact the dura mater. Since proof is ample and the electrodes are exactly the same as in previous successful studies it has been restrained from these intricate procedures and observed the animal's behavior on electrical stimulation instead. All animals showed a clear reaction in stopping their current actions and three pigs turned their head towards the visual field corresponding to the area of retinal stimulation. This could be observed repeatedly and only above 1.0 Volts which corresponds well to previous stimulation experiments Gekeler et al. (2004), loc. cit.

The documentation of behavioral changes certainly does not replace electrophysiologic techniques to provide definitive objective proof of retinal or visual pathway stimulation. However, it is believed that their highly reproducible correlation to the stimulus onset and especially their threshold dependence provide strong evidence that the animals indeed reacted to the electrical stimulus.

Light microscopy failed to demonstrate any pathologic changes except for the well-known fact that outer retinal layers degenerate because of the interruption of metabolic exchange with the RPE. However, it must be noted that these layers are the ones which degenerate in the target diseases of retinal prostheses, i.e. RP and age-related macular degeneration.

In conclusion, the study underlying this application has proven for the first time the feasibility of a compound subretinal prosthesis with permanent extra-ocular connections for energy supply. The success in freely moving research animals promises even better results in experimental implantations in humans. First, because mechanical stability will be even higher than in animals due to human physiology and cooperation of the subjects. Second, because the risk of infections will certainly be lower in humans due to hygienic protocols and higher cleanliness standards, and third the risk for PVR is expected to be lower due to the age of the subjects (inferring a slower metabolism) and greater experience in human ocular pathophysiology and wider treatment options. By providing proof of the principal feasibility of this approach of a visual prosthesis this study represents the definite last step before human trials.

## Claims

1. A retinal implant to be implanted into an eye of a mammalian for electrical stimulation of the retina, said implant comprising an inner portion to be placed into the subretinal space, preferably on the retinal pigment epithelium, an extra-ocular portion that after implantation remains outside said eye for connection to an external power source, and a connecting portion for interconnecting said inner and extra-ocular portions,
**characterized in that**
said connecting portion has attached thereto at least one patch for fixation of said connecting portion on the sclera.

2. The implant of claim 1, **characterized in that** the connecting portion is a foil, preferably made from polyimide or parylene, having attached thereto said at least one patch.

## Patentansprüche

1. Retinaimplantat zur Implantation in ein Auge eines Säugetiers für eine elektrische Stimulation der Retina, wobei das Implantat einen in dem subretinalen Raum, vorzugsweise auf dem Retina-Pigmentepithel zu platzierenden, inneren Abschnitt, einen Außeraugenabschnitt, der nach der Implantation zur Verbindung mit einer externen Energiequelle außerhalb des Auges verbleibt, sowie einen Verbindungsabschnitt umfasst, um den inneren Abschnitt und den Au-βeraugenabschnitt miteinander zu verbinden,
**dadurch gekennzeichnet, dass** an dem Verbindungsabschnitt wenigstens ein Läppchen zur Fixierung des Verbindungsabschnitts an der Sklera befestigt ist.

2. Implantat nach Anspruch 1, **dadurch gekennzeichnet, dass** der Verbindungsabschnitt eine vorzugsweise aus Polyimid oder Parylen gefertigt Folie ist, an der das wenigstens eine Läppchen befestigt ist.

## Revendications

1. Implant rétinien à implanter dans l'oeil d'un mammifère pour une stimulation électrique de la rétine, ledit implant comprenant une partie interne à placer dans l'espace sous-rétinien, de préférence sur l'épithélium de pigment rétinien, une portion extra-oculaire qui, après l'implantation, reste en dehors dudit oeil pour la connexion à une source électrique externe, et une portion de connexion pour interconnecter lesdites parties internes et extra-oculaires,
**caractérisé en ce que**
sur ladite portion de connexion est fixé au moins un patch pour la fixation de ladite partie de connexion sur le blanc de l'oeil.

2. Implant selon la revendication 1, **caractérisé en ce que** la portion de connexion est une feuille, de préférence réalisée en polyimide ou parylène, sur laquelle est fixé au moins un patch.
